# EUROPÄISCHE PATENTSCHRIFT

(11) **EP 0 462 506 B1**
(45) Veröffentlichungstag und Bekanntmachung des Hinweises auf die Patenterteilung: **06.11.1996**
(21) Anmeldenummer: 91109673.3
(22) Anmeldetag: 13.06.1991
(51) Int. Cl.: A61K 31/545

(54) **Pharmazeutische Kombinationspräparate enthaltend Cefotaxim und Xanthinderivate und deren Verwendung**
Pharmaceutical preparations of mixtures comprising cefotaxim and derivatives of xanthine and their use
Préparations pharmaceutiques de mélanges contenant cefotaxim et des dérivés de de xanthine et leur application

(30) Priorität: 20.06.1990 DE 4019571
(43) Veröffentlichungstag der Anmeldung: 27.12.1991
(73) Patentinhaber: TAIHO PHARMACEUTICAL CO., LTD., Tokyo 101 (JP)
(72) Erfinder: Klesel, Norbert, Dr., W-6103 Griesheim (DE); Limbert, Michael, Dr., W-6238 Hofheim (DE); Schrinner, Elmar, Dr., W-6200 Wiesbaden (DE)
(74) Vertreter: Weisert, Annekäte, Dipl.-Ing. Dr.-Ing.

(56) Entgegenhaltungen:
- EP-A- 0 344 586

## Beschreibung

Die vorliegende Erfindung betrifft pharmazeutische Kombinationspräparate enthaltend Cephalosporin- und Xanthinderivate und deren Verwendung zur Prophylaxe und Behandlung bakterieller Infektionskrankheiten und zur Behandlung und Prophylaxe des septischen Schocks.

Die antibiobische Wirkung von Cephalosporinen ist seit langem bekannt. Ebenfalls bekannt sind unterschiedliche Wirkungen von Xanthin-Derivaten, u.a. auch die TNF-inhibitorische Wirkung von Xanthin-Derivaten (EP 0 344 586), die die Verabreichung von Xanthin-Derivaten im Falle des septischen Schocks angezeigt sein läßt. Überraschenderweise wurde nun gefunden, daß die Wirksamkeit von Cephalosporinderivaten durch die gleichzeitige Verabreichung von Xanthinderivaten signifikant gesteigert werden kann.
Erfindungsgegenstand sind demzufolge pharmazeutische Kombinationspräparate, die mindestens ein Cephalosporin- und mindestens ein Xanthinderivat enthalten und dadurch gekennzeichnet sind, daß das Cephalosporinderivat Cefotaxim ist und das Xanthinderivat
1-(5-Hydroxyhexyl)-3-methyl-7-propylxanthin, 3-Methyl-1-(5-oxohexyl)-7-propylxanthin (= Propentofyllin), 7-Propyl- oder 7-Ethoxymethyl-1-(5-hydroxy-5-methylhexyl)-3-methylxanthin oder 1-(5-Hydroxy-5-methylhexyl)-3-methylxanthin ist.

Bevorzugt ist ein Kombinationspräparat, das Cefotaxim und 1-(5-Hydroxy-5-methylhexyl)-3-methylxanthin enthält.

Die erfindungsgemäßen Kombinationspräparate eignen sich zur Prophylaxe und Behandlung von bakteriellen Infektionskrankheiten und insbesondere zur Prophylaxe und Behandlung des septischen Schocks.

Die Herstellung und die Eigenschaften von Cephalosporinderivaten sind z.B. beschrieben in den Deutschen Offenlegungsschriften 27 02 501, 27 13 272, 27 15 385, 28 10 922, 29 21 316, 29 22 036, in der EP 00 64 740, in der GB 2 105 334 oder der GB 2 105 335. Physiologisch verträgliche Salze dieser Verbindungen sind ebenfalls in den genannten Druckschriften aufgeführt.

Die Herstellung von Xanthinderivaten ist z.B. in der DE-B-1 233 405, der DE-B-1 235 320 oder der DE 35 25 801 A1 beschrieben.

Die Cephalosporinderivate können sowohl in Kombination mit den Xanthinderivaten in getrennten Dosierungseinheiten (gleichzeitig oder in zeitlicher Abfolge) als auch mit den Xanthinderivaten vermischt verabreicht werden.

Die Herstellung erfindungsgemäßer pharmazeutischer Kombinationspräparate, die ebenfalls zum Gegenstand der vorliegenden Erfindung gehört, erfolgt, indem mindestens ein Cephalosporinderivat und mindestens ein Xanthinderivat gegebenenfalls mit weiteren Zusatz- und/oder Hilfsstoffen in eine geeignete Darreichungsform gebracht werden. Die Zusatz- oder Hilfsstoffe stammen aus der Gruppe der Trägermittel, Konservierungsstoffe und anderer üblicher Hilfsstoffe. Z.B. können für orale Darreichungsformen Hilfsstoffe wie Stärke, z.B. Kartoffel-, Mais- oder Weizenstärke, Cellulose bzw. deren Derivate, insbesondere mikrokristalline Cellulose, Siliziumdioxid, verschiedene Zucker wie Milchzucker, Magnesiumcarbonat und/oder Calciumphosphate verwendet werden. Weiterhin ist es vorteilhaft, den oralen Darreichungsformen Hilfsstoffe zuzusetzen, die die Verträglichkeit der Medikamente verbessern, wie z.B. Schleimbildner und Harze. Zwecks besserer Verträglichkeit können die Medikamente auch in Form von magensaftunlöslichen Kapseln verabreicht werden. Darüber hinaus kann es vorteilhaft sein, der

Darreichungsform, bzw. einer Komponente des Kombinationspräparats, ein Retardierungsmittel zuzusetzen, gegebenenfalls in Form von permeablen Membranen, wie z.B. solche auf Cellulose- oder Polystyrolharzbasis, oder Ionenaustauschern.

Die genannten pharmazeutischen Kombinationspräparate können auf unterschiedliche Weise appliziert werden. Z.B. können sie intravenös, intramuskulär, intraperitoneal, subkutan oder oral verabreicht werden.

Bei den erfindungsgemäßen pharmazeutischen Kombinationspräparaten kann das Verhältnis der Cephalosporinderivate zu den Xanthinderivaten einen weiten Bereich überstreichen. Zu bevorzugen ist ein Gewichtsverhältnis von ca. 1:100 bis ca. 100:1, besonders bevorzugt von ca. 1:10 bis ca. 10:1.

In dem nachfolgenden Beispiel wird die Steigerung der antibakteriellen Wirksamkeit von Cefotaxim durch die Kombination mit 1-(5-Hydroxy-5-methylhexyl)-methylxanthin (HMHM) untersucht.

### Beispiel 1

Als Versuchstiere werden NMRI-Mäuse mit einem Versuchsgewicht von 10 - 22 g verwendet. Die Tiere haben während der Versuche Zugang zu Leitungswasser und Körnerfutter ad libitum.

Die drei in dieser Studie verwendeten Bakterienstämme Staphylococcus aureus Giorgio, Escherichia coli 078 und Salmonella typhimurium MZ II zeigten in Vorversuchen bei Mäusen eine hohe Infektiosität. Suspensionen dieser Infektionserreger (in 15 % Magermilch als Gefrierschutz) werden in flüssigem Stickstoff aufbewahrt. Vor den Versuchen werden die Suspensionen in 5 %igem Mucin auf die mehrfach letalen Infektionsdosen (in Kolonie-bildenden Einheiten, KBE) eingestellt. Sie betragen 1 x 10⁶ KBE pro Maus bei der Infektion mit S. aureus Giorgio, 1 x 10⁴ KBE pro Maus bei der E. coli 078-Septikämie und 2,5 x 10³ KBE pro Maus bei der S. typhymurium MZ II-Infektion.

Die Mäuse werden intraperitoneal mit 0,3 ml Suspension der drei Bakterienstämme in 5 % Schweinemagen-Mucin infiziert.

Die Infektionsdosis enthält, je nach Keim, die 10 bis 500-fache letale Dosis an Bakterien. Je nach Infektionserreger sterben die infizierten Tiere der unbehandelten Kontrollgruppe innerhalb von sechs bis 24 Stunden nach Setzen der Infektion.

Eine Gruppe von acht infizierten Tieren dient als Infektionskontrolle. Die Tiere erhalten 0,5 ml physiologische Kochsalzlösung unmittelbar nach Setzen der Infektion.

Eine zweite Gruppe von acht infizierten Tieren wird unmittelbar nach der Infektion mit 50 mg/kg HMHM i.p. behandelt. Eine weitere Gruppe (Therapiekontrolle) von infizierten Tieren wird mit verschieden hohen Dosen von Cefotaxim (enthalten in 0,5 ml Wasser) und mit physiologischer Kochsalzlösung (0,5 ml) unmittelbar nach der Infektion i.p. behandelt.

Eine vierte Gruppe von Mäusen wird unmittelbar nach der Infektion i.p. mit verschieden hohen Cefotaxim-Konzentrationen (acht Tiere pro Cefotaxim-Konzentration; Cefotaxim-Dosis wie in Gruppe 3) und mit 50 mg/kg HMHM therapiert.

Die Zahl der überlebenden Tiere wird über 10 Tage täglich notiert. Anhand dieser Zahlen wird mit Hilfen der Probit-Methode die mittlere effektive Dosis (ED_{50%}) von Cefotaxim berechnet, d.h. diejenige Cefotaxim-Dosis, die gegeben werden muß, um das Überleben der Hälfte der Versuchstiere zu ermöglichen.

Die ED₅₀ dient als Parameter zur Bewertung der chemotherapeutischen Aktivität von Cefotaxim bzw. der Kombinationen von Cefotaxim mit HMHM.

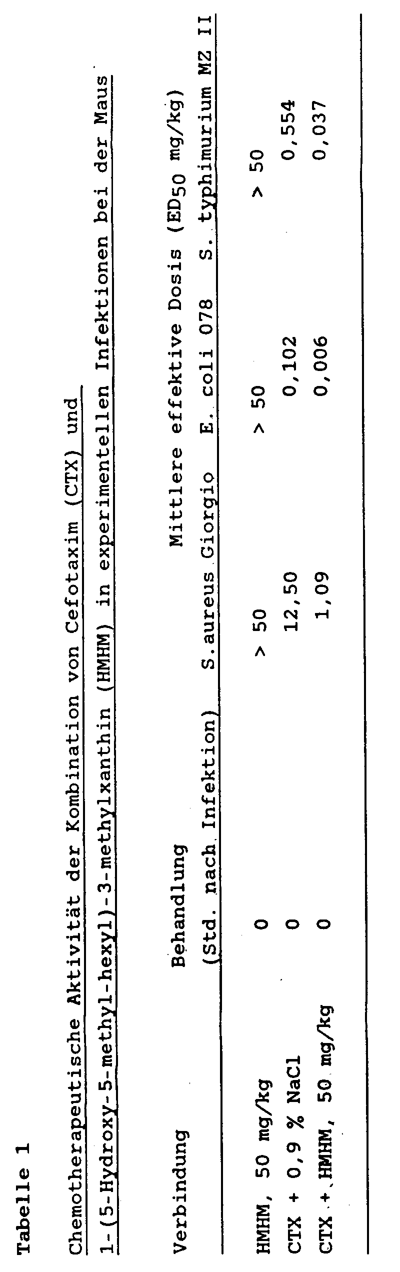

## Patentansprüche (Patentansprüche für folgende(n) Vertragsstaat(en): AT, BE, CH, DE, DK, FR, GB, IT, LI, LU, NL, SE)

1. Pharmazeutisches Kombinationspräparat, enthaltend mindestens ein Cephalosporinderivat und mindestens ein Xanthinderivat dadurch gekennzeichnet, daß das Cephalosporinderivat Cefotaxim ist und
daß das Xanthinderivat 1-(5-Hydroxyhexyl)-3-methyl-7-propylxanthin, 3-Methyl-1-(5-oxohexyl)-7-propylxanthin (= Propentofyllin), 7-Propyl- oder 7-Ethoxymethyl-1-(5-hydroxy-5-methylhexyl)-3-methylxanthin oder 1-(5-Hydroxy-5-methylhexyl)-3-methylxanthin ist.

2. Pharmazeutisches Kombinationspräparat gemäß Anspruch 1, dadurch gekennzeichnet, daß es Cefotaxim und 1-(5-Hydroxy-5-methylhexyl)-3-methylxanthin enthält.

3. Verwendung von Cefotaxim und mindestens einem Xanthinderivat gemäß Anspruch 1 oder 2 zur Herstellung von Arzneimitteln.

4. Verfahren zur Herstellung eines pharmazeutischen Kombinationspräparates gemäß Anspruch 1 oder 2, dadurch gekennzeichnet, daß Cefotaxim und mindestens ein Xanthinderivat gemäß Anspruch 1 oder 2 gegebenenfalls mit geeigneten Hilfs- und/oder Trägerstoffen in eine geeignete Darreichungsform gebracht werden.

5. Verwendung von Cefotaxim und mindestens einem Xanthinderivat gemäß Anspruch 1 oder 2 zur Herstellung eines Arzneimittels zur Prophylaxe oder zur Behandlung von bakteriellen Infektionskrankheiten.

6. Verwendung von Cefotaxim und mindestens einem Xanthinderivat gemäß Anspruch 1 oder 2 zur Herstellung eines Arzneimittels zur Prophylaxe oder zur Behandlung des septischen Schocks.

## Patentansprüche (Patentansprüche für folgende(n) Vertragsstaat(en): ES, GR)

1. Verfahren zur Herstellung eines pharmazeutischen Kombinationspräparates, dadurch gekennzeichnet, daß mindestens ein Cephalosporinderivat und mindestens ein Xanthinderivat gegebenenfalls mit geeigneten Hilfs- und/oder Trägerstoffen in eine geeignete Darreichungsform gebracht werden dadurch gekennzeichnet, daß das Cephalosporinderivat Cefotaxim ist und
daß das Xanthinderivat 1-(5-Hydroxyhexyl)-3-methyl-7-propylxanthin, 3-Methyl-1-(5-oxohexyl)-7-propylxanthin (= Propentofyllin), 7-Propyl- oder 7-Ethoxymethyl-1-(5-hydroxy-5-methylhexyl)-3-methylxanthin oder 1-(5-Hydroxy-5-methylhexyl)-3-methylxanthin ist.

2. Verfahren gemäß Ansprüch 1, dadurch gekennzeichnet, daß das Kombinationspräparat Cefotaxim und 1-(5-Hydroxy-5-methylhexyl)-3-methylxanthin enthält.

3. Verwendung von Cefotaxim und mindestens einem Xanthinderivat gemäß Anspruch 1 oder 2 zur Herstellung von Arzneimitteln.

4. Verwendung von Cefotaxim und mindestens einem Xanthinderivat gemäß Anspruch 1 oder 2 zur Herstellung eines Arzneimittels zur Prophylaxe oder zur Behandlung von bakteriellen Infektionskrankheiten.

5. Verwendung von Cefotaxim und mindestens einem Xanthinderivat gemäß Anspruch 1 oder 2 zur Herstellung eines Arzneimittels zur Prophylaxe oder zur Behandlung des septischen Schocks.

## Claims (Claims for the following Contracting State(s): AT, BE, CH, DE, DK, FR, GB, IT, LI, LU, NL, SE)

1. A pharmaceutical combination preparation, containing at least one cephalosporine derivative and at least one xanthine derivative, characterised in that the cephalosporine derivative is cefotaxime and the xanthine derivative is 1-(5-hydroxyhexyl)-3-methyl-7-propylxanthine, 3-methyl-1-(5-oxohexyl)-7-propylxanthine (= propentofyllin), 7-propyl- or 7-ethoxymethyl-1-(5-hydroxy-5-methylhexyl)-3-methylxanthine or 1-(5-hydroxy-5-methylhexyl)-3-methylxanthine.

2. A pharmaceutical combination preparation in accordance with Claim 1, characterised in that it contains cefotaxime and 1-(5-hydroxy-5-methylhexyl)-3-methylxanthine.

3. Use of cefotaxime and at least one xanthine derivative in accordance with Claim 1 or 2 to prepare medicinal drugs.

4. A process for preparing a pharmaceutical combination preparation in accordance with Claim 1 or 2, characterised in that cefotaxime and at least one xanthine derivative in accordance with Claim 1 or 2, optionally with appropriate auxiliary substances and/or carriers, are combined in a suitable form of presentation.

5. Use of cefotaxime and at least one xanthine derivative in accordance with Claim 1 or 2 to prepare a medicinal drug for prophylaxis or treatment of bacterial infections.

6. Use of cefotaxime and at least one xanthine derivative in accordance with Claim 1 or 2 to prepare a medicinal drug for prophylaxis or treatment of endotoxin shock.

## Claims (Claims for the following Contracting State(s): ES, GR)

1. A process for preparing a pharmaceutical combination preparation, characterised in that at least one cephalosporine derivative and at least one xanthine derivative, optionally with appropriate auxiliary substances and/or carriers, are combined in a suitable form of presentation, characterised in that the cephalosporine derivative is cefotaxime and the xanthine derivative is 1-(5-hydroxyhexyl)-3-methyl-7-propylxanthine, 3-methyl-1-(5-oxohexyl)-7-propylxanthine (= propentofyllin), 7-propyl- or 7-ethoxymethyl-1-(5-hydroxy-5-methylhexyl)-3-methylxanthine or 1-(5-hydroxy-5-methylhexyl)-3-methylxanthine.

2. A process in accordance with Claim 1, characterised in that the combination preparation contains cefotaxime and 1-(5-hydroxy-5-methylhexyl)-3-methylxanthine.

3. Use of cefotaxime and at least one xanthine derivative in accordance with Claim 1 or 2 to prepare medicinal drugs.

4. Use of cefotaxime and at least one xanthine derivative in accordance with Claim 1 or 2 to prepare a medicinal drug for prophylaxis or treatment of bacterial infections.

5. Use of cefotaxime and at least one xanthine derivative in accordance with Claim 1 or 2 to prepare a medicinal drug for prophylaxis or treatment of endotoxin shock.

## Revendications (Revendications pour l'(les) Etat(s) contractant(s) suivant(s): AT, BE, CH, DE, DK, FR, GB, IT, LI, LU, NL, SE)

1. Association pharmaceutique, contenant au moins un dérivé de céphalosporine et au moins un dérivé de xanthine, caracterisée en ce que le dérivé de céphalosporine est le céfotaxime et que le dérivé de xanthine est la 1-(5-hydroxyhexyl)-3-méthyl-7-propylxanthine, la 3-méthyl-1-(5-oxohexyl)-7-propylxanthine (= propentofylline) 7-propyl ou 7-éthoxyméthyl-1-(5-hydroxy-5-méthylhexyl)-3-méthylxanthine ou la 1-(5-hydroxy-5-méthylhexyl)-3-méthylxanthine.

2. Association pharmaceutique selon la revendication 1, caractérisée en ce qu'elle contient du céfotaxime et de la 1-(5-hydroxy-5-méthylhéxyl)-3-méthylxanthine.

3. Utilisation de céfotaxime et d'au moins un dérivé de xanthine selon la revendication 1 ou 2 pour la préparation de médicaments.

4. Procédé de préparation d'une association pharmaceutique selon la revendication 1 ou 2, caractérisé en ce que le céfotaxime et au moins un dérivé de xanthine selon la revendication 1 ou 2, accompagnés le cas échéant d'adjuvants et/ou de véhicules appropriés, sont amenés à une forme pharmaceutique appropriée.

5. Utilisation de céfotaxime et d'au moins un dérivé de xanthine selon la revendication 1 ou 2 pour la préparation d'un médicament destiné à la prévention ou au traitement de maladies infectieuses bactériennes.

6. Utilisation de céfotaxime et d'au moins un dérivé de xanthine selon la revendication 1 ou 2 pour la préparation d'un médicament destiné à la prévention ou au traitement du choc septique.

## Revendications (Revendications pour l'(les) Etat(s) contractant(s) suivant(s): ES, GR)

1. Procédé de préparation d'une association pharmaceutique, caractérisé en ce qu'au moins un dérivé de céphalosporine et au moins un dérivé de xanthine, accompagnés le cas échéant d'adjuvants et/ou de véhicules appropriés, sont amenés à une forme pharmaceutique appropriée, caractérisé en ce que le dérivé de céphalosporine est le céfotaxime et que le dérivé de xanthine est la 1-(5-hydroxyhexyl)-3-méthyl-7-propylxanthine, la 3-méthyl-1-(5-oxohexyl)-7-propylxanthine (= propentofylline) 7-propyl ou 7-éthoxyméthyl-1-(5-hydroxy-5-méthylhexyl)-3-méthylxanthine ou la 1-(5-hydroxy-5-méthylhexyl)-3-méthylxanthine.

2. Procédé selon la revendication 1, caractérisé en ce que l'association contient du céfotaxime et de la 1-(5-hydroxy-5-méthylhexyl)-3-méthylxanthine.

3. Utilisation de céfotaxime et d'au moins un dérivé de xanthine selon la revendication 1 ou 2 pour la préparation de médicaments.

4. Utilisation de céfotaxime et d'au moins un dérivé de xanthine selon la revendication 1 ou 2 pour la préparation d'un médicament destiné à la prévention ou au traitement de maladies infectieuses bactériennes.

5. Utilisation de céfotaxime et d'au moins un dérivé de xanthine selon la revendication 1 ou 2 pour la préparation d'un médicament destiné à la prévention ou au traitement du choc septique.
